Europäisches Patentamt

European Patent Office

Office européen des brevets

(11) Veröffentlichungsnummer: **0 212 374**
**B1**

(12) **EUROPÄISCHE PATENTSCHRIFT**

(45) Veröffentlichungstag der Patentschrift: **14.11.90**

(21) Anmeldenummer: **86110551.8**

(22) Anmeldetag: **30.07.86**

(51) Int. Cl.⁵: **C 07 H 5/02**, C 07 H 15/18, C 07 H 15/04

(54) **Verfahren zur Herstellung von alkylierten hydroxylgruppenfreien Glycosylfluoriden.**

(30) Priorität: **09.08.85 DE 3528654**

(43) Veröffentlichungstag der Anmeldung:
**04.03.87 Patentblatt 87/10**

(45) Bekanntmachung des Hinweises auf die
Patenterteilung:
**14.11.90 Patentblatt 90/46**

(84) Benannte Vertragsstaaten:
**AT BE CH DE FR GB IT LI NL SE**

(56) Entgegenhaltungen:
**CHEMICAL ABSTRACTS, Band 104, Nr. 21, 26.
Mai 1986, Seite 664, Zusammenfassung Nr.
186733k, Columbus, Ohio, US; V.S. ABRAMOV
et al.: "Synthesis of benzylated glycosyl
fluorides", & ZH. OBSHCH. KHIM. 1985, 55(8),
1885-6**

**M. WINDHOLZ et al.: "The Merck Index", 10.
Ausgabe, Seite ONR-96, Merck & Co., Inc,
Rahway, N.J., US; A.W. WILLIAMSON:
"Williamson Synthesis"**

(73) Patentinhaber: **HOECHST
AKTIENGESELLSCHAFT
Postfach 80 03 20
D-6230 Frankfurt am Main 80 (DE)**

(72) Erfinder: **Thiem, Joachim, Prof. Dr.
Asbeckweg 31
D-4400 Münster (DE)**
Erfinder: **Deger, Hans-Matthias, Dr.
Am Rheingauer Weg 8
D-6238 Hofheim am Taunus (DE)**
Erfinder: **Fritsche-Lang, Wolfram, Dr.
Rhönstrasse 7
D-6140 Bensheim (DE)**
Erfinder: **Kreuzer, Matthias
Im Münchfeld 8
D-6500 Mainz (DE)**

(56) Entgegenhaltungen:
**JOURNAL OF THE CHEMICAL SOCIETY,
CHEMICAL COMMUNICATIONS, Nr. 17, 1984,
Seiten 1155-1156, The Chemical Society, GB;
K.C. NICOLAOU et al.: "Reactions of glycosyl
fluorides. Synthesis of O-, S-, and N-glycosides"**

**CHEMISTRY LETTERS, 1984, Seiten 1747-1750,
The Chemical Society of Japan, JP; M. HAYASHI
et al.: "Simple synthesis of glycosyl fluorides"**

Courier Press, Leamington Spa, England.

**Beschreibung**

Von den Glycosylhalogeniden wurden bisher praktisch nur die Chlor- und Bromverbindungen in Form der geschützten Derivate untersucht und in der Kohlenhydratchemie eingesetzt. Doch selbst diese zeigen nur eingeschränkte Stabilität, was durch ihre Reaktion mit Basen—wie Diäthylamin—in aprotischen Lösungsmitteln zu acylierten Hydroxyglycalen gezeigt werden kann (R. J. Ferrier, Meth. Carbohydr. Chem. 6, 307 (1972)).

Von den ungeschützten Glycosylhalogeniden sind lediglich die Glycosylfluoride, insbesondere die α-Glycosylfluoride, relativ stabil. An diesen durchgeführte kinetische Untersuchungen ergaben allerdings auch, daß sowohl unter Basen-, besonders aber bei Säure-Katalyse eine hydrolytische Zersetzung erfolgt (J. E. G. Barnett, Carbohydr. Res. 9, 21 (1969).

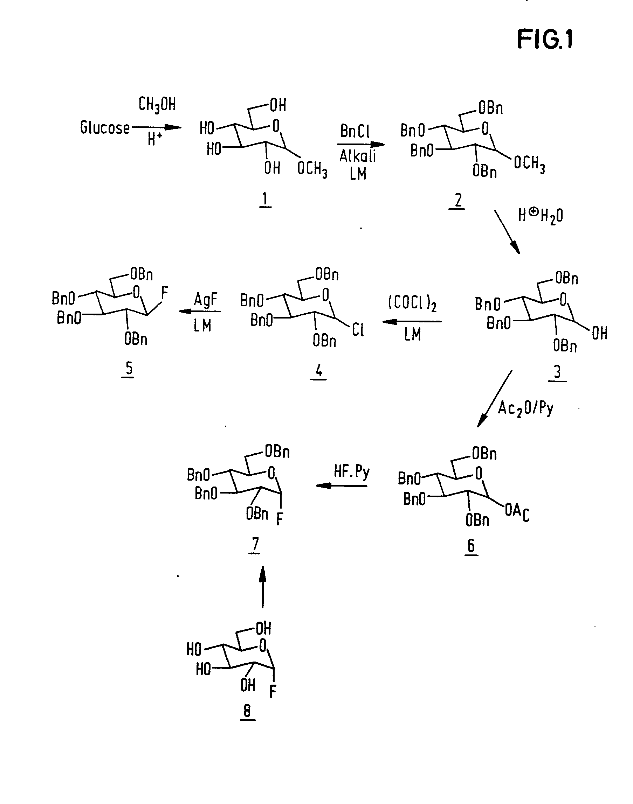

Für den Aufbau komplex-chiraler Verbindungen wie niederer Oligosaccharide kommt der Verfügbarkeit peralkylierter Glycosylfluoride als Glycosylierungsmittel besonderes Interesse zu, dan inzwischen einige neue Verfahren für deren Verwendung in Glycosylierungsreaktionen entwickelt wurden (Deutsche Patentanm. P 34 26 074.9 (1984)). Von dieser Substanzklasse sind bisher nur die beiden anomeren Fluoride 5 und 7 (s. Figur 1) bekannt, deren mehrstufige Synthesen aus Glucose dort aufgeführt sind. Für die Herstellung von 5 wird das aus Glucose zugängliche α-Methylglucosid 1 durch Williamson'sche Äthersynthese zu dem Tetra-0-benzyl-Derivat 2 umgesetzt, welches über die Stufen der C-1-Hydrolyse, C-1-Chlorierung und C-1-Fluoridierung mit Silberfluorid in das gewünschte 5 übergeführt wird, (C. P. J. Glaudemans und H. G. Fletcher Jr., Meth. Carbohydr. Chem. 6, 373 (1972); J. R. Pougny, M. A. N. Nassr, N. Naulet und P. Sinay, Nouv. J. Chem. 2, 389 (1978); T. Mukaiyama, Y. Murai und S. Shoda, Chem. Lett. 1981, 431. Über die Acetylierung von 3 zum α-Acetat 6 und nachfolgende Behandlung mit dem Pyridin-Fluorwasserstoff-Addukt (Olah's Reagenz) ist die Herstellung des entsprechenden 7 beschrieben (S. Hashimoto, M. Hayashi und R. Noyori, Tetrahedron Lett. 25, 1379 (1984)).

Überraschend wurde nun gefunden, daß die ungeschützten Glycosylfluoride in dipolar-aprotischen Lösungsmitteln erstaunlich basenstabil sind. Dies eröffnet die Möglichkeit für einen neuartigen Zugang zu hydroxylgruppenfreien alkylierten, insbesondere peralkylierten Glycosylfluoriden.

Gegenstand der Erfindung ist somit ein Verfahren zur Herstellung von hydroxylgruppenfreien alkylierten Glycosylfluoriden, das dadurch gekennzeichnet ist, daß man Glycosylfluoride, die noch Hydroxylgruppen enthalten, (nach der Art der Williamson'schen Äthersynthese) in Gegenwart von basischen Systemen von Oxiden und/oder Hydroxiden der 1. Gruppe des Periodensystems, d.h. Alkalihydroxide wie Natriumhydroxid, Kaliumhydroxid oder Oxide wie Silberoxid, sowie von Alkalialkoholaten, deren Alkoholkomponente bis zu 6, insbesondere bis zu 4 C-Atome hat, wie Kalium-tert.-butylat, und von Alkalihydriden, wie Natriumhydrid, mit "Alkylierungsmitteln" in die "alkylierten" Verbindungen überführt (Methodik bei R. E. Wing und J. N. Bemiller Meth. Carbohydr. Chem. 6, 368 (1972)). Der Begriff "alkyliert" bedeutet dabei und im folgenden, daß durch das "Alkylierungsmittel" in die Ätherbindungen ein gesättigtes aliphatisches Kohlenstoffatom eingeführt wird, an das seinerseits entweder Wasserstoff oder weitere Kohlenwasserstoffreste gebunden sind. Als Alkylierungsmittel kommen bevorzugt die Halogen-Derivate zum Einsatz, insbesondere die entsprechenden Chlor-, Brom- und Jodverbindungen, wie Methyljodid. Es können allerdings auch die Dialkylsulfate vorteilhaft eingesetzt werden. Mit anderen Worten kommen als Ätherreste in Frage: Methyl oder in Kombination mit gesättigten Kohlenwasserstoffresten Äthyl, n-Propyl, Isopropyl, die verschiedenen Butyle, Hexyle, Octyle, Dodecyle, Hexadecyle, Octadecyle oder Verbindungen mit $C_{20}$, wie das 2-Äthylhexyl, Lauryl, Palmityl oder Stearyl; in Kombination mit ungesättigten Kohlenwasserstoffresten sind darunter z.B. zu verstehen Allyl, Crotyl, Diisobutenyl, Oleyl, sowie Benzyl, Benzhydryl, Trityl, ferner auch mehrkernige Systeme wie —$CH_2$-Naphthyl bis zu einer Gesamt-Kohlenstoffatom-Anzahl von 25, die jeweils am aromatischen Kern noch Reste mit ein oder mehreren elektronenziehenden oder -schiebenden Substituenten wie Methoxy, Methyl, Halogen, Nitro- oder Nitril-Gruppen enthalten können.

Bevorzugt wird das Verfahren in einem dipolar-aprotischen Lösungsmittel wie Dimethylformamid, Dimethylsulfoxid, Hexamethylphosphorsäuretriamid (HMPT), Dimethylpropylenharnstoff (DMPU) durchgeführt. Andere Basen wie Calciumoxid, Kaliumcarbonat oder 1,5-Diazabicyclo-[5,4,0]-undecen-5 (DBU) sind nicht wirksam.

Das erfindungsgemäße Verfahren stellt somit eine einfache einstufige Methode zur Herstellung von insbesondere peralkylierten Glycosylfluoriden dar, die als Ausgangsverbindungen für die Gewinnung von Glycosiden geeignet sind (Deutsche Patentanm. P 34 26 074.9 (1984)).

Für das erfindungsgemäße Verfahren werden als Ausgangsverbindungen im allgemeinen solche Glycosylfluoride verwendet, deren sämtliche Hydroxylgruppen noch unsubstituiert sind. Jedoch ist es auch möglich, partiell acylierte Glycosylfluoride zu verwenden, so daß man gemischt acylierte und alkylierte Glycosylfluoride erhält. Für die Umsetzung solcher partiell acylierter Glycosylfluoride hat sich Silberoxid als Base besonders bewährt. Derartige Acylalkylglycosylfluoride sind für den Aufbau komplex verzweigter niederer Oligosaccharide geeignet.

Geeignete Ausgangsverbindungen sind neben α-Glucosylfluorid auch die α-Fluoride anderer reduzierender Mono- bzw. Oligosaccharide—wobei dieser Begriff 2—4 Struktureinheiten umfaßt—in Frage, wie die α-Fluoride von Galactose, Mannose, Gulose, Talose, Allose, Altrose usw. einschließlich der α-Fluoride aus der Reihe der $C_5$-Aldosen wie Xylose, Ribose usw. bzw. bei den Oligosacchariden

insbesondere die α-Fluoride der Disaccharide wie Lactose, Maltose, Cellobiose, Gentiobiose usw. oder auch höherer Saccharide wie Maltotriose, Raffinose, Maltotetraose und Stachyose. Auch auf die Fluoride der N-Acylamino-desoxyzucker wie N-Acetylamino-glucopyranosylfluorid ist das erfindungsgemäße Verfahren anwendbar.

Durch die Anwendung des erfindungsgemäßen Verfahrens können so u.a. in ausgezeichneter Ausbeute die kristallinen, bisher zum Teil nicht beschriebenen Verbindungen 2,3,4,6-Tetra-0-benzyl-α-D-galactopyranosylfluorid (10), 2-Acetamido-3,4,6-tri-0-benzyl-2-desoxy-α-D-glucopyranosylfluorid (12), 2,3,6-Tri-0-benzyl-4-0(2,3,4,6-tetra-0-benzyl-β-D-galactopyranosyl)-α-D-glucopyranosylfluorid (14) und 2,3,4,6-Tetra-0-methyl-α-D-glucopyranosylfluorid (15) erhalten werden.

Die erfindungsgemäße Reaktion wird vorteilhaft zwischen −20°C und +40°C, bevorzugt zwischen −5°C und +25°C durchgeführt und im allgemeinen bei Atmosphärendruck. Die Anwendung von Über- oder Unterdruck ist möglich, aber nicht bevorzugt.

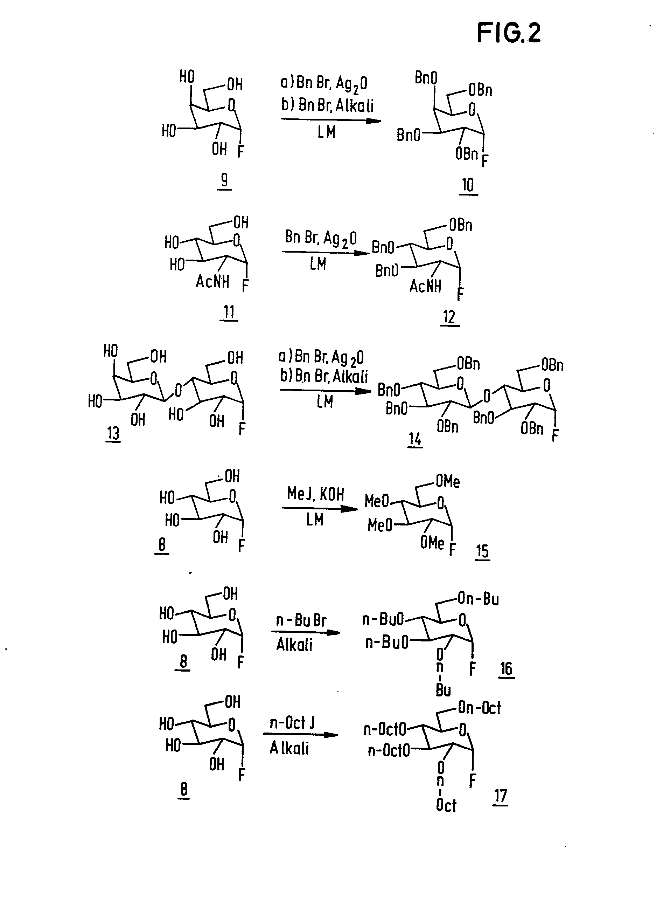

In den folgenden Beispielen beziehen sich die Lösungsmittelverhältnisse jeweils auf das Volumen. In den Figuren 1 und 2 bedeutet Bn Benzyl, LM Lösungsmittel, Py Pyridin, Me Methyl, n-BuBr 1-Bromobutan und n-Oct J 1-Jodoctan.

Beispiele:
1. 2,3,4,6-Tetra-0-benzyl-α-D-glucopyranosylfluoride (7):
a) 2,6 g (14,3 mmol) 8 wurden in 50 ml wasserfreiem Dimethylformamid gelöst und mit 26,5 g (114 mmol) frisch hergestelltem Silberoxid und 14 ml (20 g, 118 mmol) Benzylbromid bei Raumtemperatur gerührt. Nach einer Stunde war die Reaktion beendet, wie sich durch Dünnschichtchromatographie in Methylenchlorid/Diäthyläther (Äther) (20:1) feststellen läßt. Die Lösung wurde über ein Silikatfilter-Hilfsmittel (®Celite) filtriert und im Hochvakuum eingeengt. Das Rohprodukt konnte durch Kristallisation aus Äther oder durch Säulenchromatographie (n-Hexan/Äther, 5:1) gereinigt werden. Ausbeute 6,5 g (85%).

b) 2,0 g (11 mmol) 8 wurden in 20 ml absolutem Dimethylformamid gelöst und bei 0°C mit 7,4 g (132 mmol) gepulvertem Kaliumhydroxid und 15 ml Benzylbromid versetzt. Man ließ auf Raumtemperatur erwärmen und rührte noch 3 Stunden Dann wurde die Reaktionsmischung mit 15 ml Methanol 30 min gerührt, anschließend auf Eiswasser gegossen, zweimal mit je 50 ml Dichlormethan extrahiert, über Magnesiumsulfat getrocknet, filtriert und im Hochvakuum eingeengt. Der Rückstand kristallisierte hervorragend aus n-Hexan/Äther; Ausbeute 5,2 g (87%).
Schmp. 70,5°C, $[\alpha]_D^{20}$=4,2 (c=0,87 in CHCl$_3$). $^1$H-NMR
(CDCl$_3$): δ=5,55 (dd, 1-H), J$_{1,2}$=2,5, J$_{1,F}$=52,9 Hz.

2) 2,3,4,6-Tetra-0-benzyl-α-D-galactopyranosylfluorid (10):
a) 2,6 g (14,3 mmol) 9 wurden unter den gleichen Bedingungen wie im Beispiel 1a umgesetzt und aufgearbeitet. Die neue Verbindung 10 wird in einer Ausbeute von 5,7 g (74%) erhalten.
b) 1,82 g (10,0 mmol) 9 wurden wie im Beispiel 1b umgesetzt und aufgearbeitet; Ausbeute 4,0 g (75%).
Schmp. 71°C; Sirup $[\alpha]_D^{20}$=+3,9 (c=1,14 in CHCl$_3$). $^1$H-NMR
(C$_6$D$_6$): δ=5,68 (dd, 1-H), J$_{1,2}$=2,7, J$_{1,F}$=53,9 Hz.

3) 2-Acetamido-3,4,6-tri-0-benzyl-2-desoxy-α-D-glucopyranosylfluorid (12):
Eine Lösung von 2,85 g (14,3 mmol) 11 wurde wie im Beispiel 1a umgesetzt und aufgearbeitet. Ausbeute 5,9 g (77%),
Schmp. 188°C, $[\alpha]_D^{20}$=+81,8 (c=0,92 in CHCl$_3$).
$^1$H-NMR (CDCl$_3$): δ=5,61 (dd, 1-H), J$_{1,2}$=2,4, J$_{1,F}$=53,0 Hz.

4) 2,3,6-Tri-0-benzyl-4-0(2,3,4,6-tetra-0-benzyl-β-D-galactopyranosyl)-α-D-glucopyranosylfluorid (14):
a) Die Lösung von 1,07 g (3,1 mmol) 13 in 30 ml absolutem Dimethylformamid wurde bei 0°C mit 10,0 g (43 mmol) Silberoxid und 5 ml (43 mmol) Benzylbromid versetzt. Man ließ auf Raumtemperatur erwärmen und rührte noch 2 Stunden. Dann wurde über Kieselgel filtriert und im Hochvakuum eingeengt. Der Rückstand wurde an einer kurzen Kieselgelsäule mit n-Hexan/Äther (3:1) gereinigt. Ausbeute 2,2 g (72%).
b) 1,72 g (5,0 mmol) 13 werden wie im Beispiel 1b umgesetzt und aufgearbeitet; Ausbeute 3,46 g (71%).
Schmp. 81°C, $[\alpha]_D^{20}$=−0,3 (c=6,33 in CHCl$_3$).
$^1$H-NMR (CDCl$_3$): δ=5,49 (dd, 1-H), J$_{1,2}$=2,6, J$_{1,F}$=53,0 Hz, 4,70 (d, 1'-H), J$_{1',2'}$=7,6 Hz.

5) 2,3,4,6-Tetra-0-methyl-α-D-glucopyranosylfluorid (15):
500 mg (2,75 mmol) 8 wurden in 10 ml wasserfreiem Dimethylformamid gelöst und bei Raumtemperatur mit 1,25 g (22,3 mmol) gepulvertem Kaliumhydroxid und 1,4 ml (22,3 mmol) Methyljodid versetzt. Nach 1 Stunde bei Raumtemperatur wird auf 0°C gekühlt, mit 5 ml Methanol versetzt und 30 min gerührt. Dann wird die Mischung auf Eiswasser gegossen zweimal mit 50 ml Dichlormethan extrahiert, über Magnesiumsulfat getrocknet und eingeengt. Die reine Verbindung wird durch chromatographische Reinigung an Kieselgel mit Toluol/Essigester, 2:1 gewonnen; Ausbeute 523 mg (80%), viskose Flüssigkeit, $[\alpha]_D^{20}$=+97,3 (c=2,72 in (CHCl$_3$).
$^1$H-NMR (CDCl$_3$): δ=5,37 (dd, 1-H), J$_{1,2}$=2,7, J$_{1,F}$=53,1 Hz.

EP 0 212 374 B1

6) 2,3,4,6-Tetra-0-n-butyl-α-D-glucopyranosylfluorid (16):

0,5 g (2,7 mmol) α-D-Glucopyranosylfluorid (8) werden in 10 ml wasserfreiem Dimethylformamid gelöst und mit 1,8 g (33 mmol) gepulvertem Kaliumhydroxid und 4,5 g (3 ml, 33 mmol) 1-Brombutan versetzt. Nach 3 Stunden bei 70°C (Wasserbad) werden 10 ml Methanol zugegeben und weitere 30 min gerührt. Die Lösung wird nach dem Abkühlen auf Eiswasser gegossen, die wässrige Phase dreimal mit Methylenchlorid extrahiert und die Extrakte über Magnesiumsulfat getrocknet. Nach dem Einengen im Hochvakuum erhält man 0,8 g eines braunen Sirups, der chromatographisch gereinigt wird (Toluol/Essigester, 20:1). Ausbeute 520 mg (42%), farbloser Sirup,
$[\alpha]_D^{20}=+61,7$ (c=0,92, CHCl$_3$),
$^1$H-NMR (C$_6$D$_6$): δ=5,61 (dd, 1-H), J$_{1,2}$=2,6, J$_{1,F}$=53.9 Hz.

7) 2,3,4,6-Tetra-0-n-octyl-α-D-glucopyranosylfluorid (17):

260 mg (1,4 mmol) α-D-Glucopyranosylfluorid (8) werden in 10 ml wasserfreiem Dimethylformamid gelöst und mit 0,95 g (17 mmol) gepulvertem Kaliumhydroxid und 3,1 ml (4,1 g, 17 mmol) 1-Jodoctan auf 70°C erhitzt. Nach 6 h werden 5 ml Methanol zugegeben und weitere 30 min ohne Erhitzen gerührt. Die Reaktionsmischung wird nach dem Abkühlen auf Eiswasser gegossen, die wässrige Phase dreimal mit Methylenchlorid extrahiert. Die über Magnesiumsulfat getrockneten Extrakte werden eingeengt und ergeben 490 mg eines Rohproduktes, das chromatographisch (Toluol/Essigester, 20:1) gereinigt wird. Ausbeute 305 mg (34%), farbloser Sirup,
$[\alpha]_D^{20}=+32,8$ (c=1,27 CHCl$_3$),
$^1$H-NMR (C$_6$D$_6$): δ=5,66 (dd, 1-H), J$_{1,2}$=2,6, J$_{1,F}$=53.6 Hz.

## Patentansprüche

1. Verfahren zur Herstellung von hydroxylgruppenfreien alkylierten Glycosylfluoriden, dadurch gekennzeichnet, daß man Glycosylfluoride, die noch Hydroxylgruppen enthalten, mit solchen Alkylierungsmitteln, die eine Ätherbindung zwischen einem gesättigten aliphatischen Kohlenstoffatom und Sauerstoff herstellen können, in Gegenwart von basischen Systemen von Oxyden und/oder Hydroxyden der ersten Gruppe des periodischen Systems und/oder von Alkalialkoholaten, deren Alkoholkomponente bis zu 6 C-Atome hat, oder von Alkalihydriden alkyliert.

2. Verfahren nach Anspruch 1, dadurch gekennzeichnet, daß das Alkylierungsmittel an dem gesättigten C-Atom noch wenigstens einen aromatischen Rest enthält, vorzugsweise eine Benzylverbindung ist.

3. Verfahren nach Anspruch 1, dadurch gekennzeichnet, daß das Alkylierungsmittel eine Chlor-, Brom- oder Jodverbindung eines aliphatischen Kohlenwasserstoffrestes ist, der gegebenenfalls bis zu einer olefinischen Doppelbindung enthält.

4. Verfahren nach einem oder mehreren der Ansprüche 1 bis 3, dadurch gekennzeichnet, daß die Reaktion in Gegenwart von Silberoxyd oder einem Alkalihydroxyd, vorzugsweise Kaliumhydroxyd durchgeführt wird.

5. Verfahren nach einem der mehreren der Ansprüche 1 bis 4, dadurch gekennzeichnet, daß die Reaktion in einem dipolaraprotischen Lösungsmittel, insbesondere Dimethylformamid durchgeführt wird.

6. Verfahren nach einem oder mehreren der Ansprüche 1 bis 5, dadurch gekennzeichnet, daß die Reaktion bei −20 bis +40, vorzugsweise bie −5 bis +25°C durchgeführt wird.

7. Verfahren nach einem oder mehreren der Ansprüche 1 bis 6, dadurch gekennzeichnet, daß in dem als Ausgangsmaterial verwendeten Glycosylfluorid sämtliche OH-Gruppen unsubstituiert sind.

8. Verfahren nach einem oder mehreren der Ansprüche 1 bis 7, dadurch gekennzeichnet, daß das als Ausgangsmaterial verwendete Glycosylfluorid partiell acyliert ist.

9. Als neue Verbindungen das 2-Acetamido-3,4,6-tri-0-benzyl-2-desoxy-α-D-glucopyranosylfluorid, das 2,3,4,6-Tetra-0-n-butyl-α-D-glucopyranosylfluorid und das 2,3,4,6-Tetra-0-n-octyl-α-D-glucopyranosylfluorid.

## Revendications

1. Procédé pour préparer des fluorures de glycosyles alkylés dépourvus de radical hydroxy, procédé caractérisé en ce qu'on alkyle des fluorures de glycosyles contenant encore des radicaux hydroxy, avec des agents d'alkylation capables de créer une liaison éther entre un atome de carbone aliphatique saturé et un atome d'oxygène, en présence de systèmes basiques d'oxydes et/ou d'hydroxydes d'éléments appartenant au premier groupe de la classification périodique, et/ou d'alcoolates de métaux alcalins dont la composante alcool renferme au plus 6 atomes de carbone, ou d'hydrures de métaux alcalins.

2. Procédé selon la revendication 1, caractérisé en ce que l'agent d'alkylation contient encore, sur l'atome de carbone saturé, au moins un radical aromatique, et est de préférence un composé benzylique.

3. Procédé selon la revendication 1, caractérisé en ce que l'agent d'alkylation est un dérivé chloré, bromé ou iodé d'un hydrocarbure aliphatique qui contient éventuellement au plus une double liaison éthylénique.

4

**EP 0 212 374 B1**

4. Procédé selon l'une quelconque des revendications 1 à 3, caractérisé en ce que la réaction est effectuée en présence d'oxyde d'argent ou d'un hydroxyde de métal alcalin, de préférence d'hydroxyde de potassium.

5. Procédé selon l'une quelconque des revendications 1 à 4, caractérisé en ce que la réaction est effectuée dans un solvant aprotique dipolaire, plus spécialement dans le diméthylformamide.

6. Procédé selon l'une quelconque des revendications 1 à 5, caractérisé en ce que la réaction est effectuée à une température comprise entre −20 et +40°C, de préférence entre −5 et +25°C.

7. Procédé selon l'une quelconque des revendications 1 à 6, caractérisé en ce que, dans le fluorure de glycosyle utilisé comme corps de départ, aucun des radicaux OH n'est substitué.

8. Procédé selon l'une quelconque des revendications 1 à 7, caractérisé en ce que le fluorure de glycosyle utilisé comme corps de départ est partiellement acylé.

9. En tant que composés nouveaux, le fluorure d'acétylamino-2 tri-0-benzyl-3,4,6 désoxy-2 α-D-glucopyranosyle, le fluorure de tétra-0-n-butyl-2,3,4,6 α-D-glucopyrannosyle et le fluorure de tétra-0-n-octyl-2,3,4,6 α-D-glucopyrannosyle.

**Claims**

1. A process for the preparation of alkylated glycosyl fluorides free from hydroxyl groups, which comprises alkylating glycosyl fluorides which still contain hydroxyl groups with alkylating agents capable of producing an ether linkage between a saturated aliphatic carbon atom and oxygen, in the presence of basic systems of oxides and/or hydroxides of the first group of the periodic system and/or alkali metal alcoholates in which the alcohol component has up to 6 carbon atoms, or of alkali metal hydrides.

2. The process as claimed in claim 1, wherein the alkylating agent still contains at least one aromatic radical on the saturated carbon atom and is preferably a benzyl compound.

3. The process as claimed in claim 1, wherein the alkylating agent is a chlorine, bromine, or iodine compound of an aliphatic hydrocarbon radical which contains, if appropriate, up to one olefinic double bond.

4. The process as claimed in one or more of claims 1 to 3, wherein the reaction is carried out in the presence of silver oxide or an alkali metal hydroxide, preferably potassium hydroxide.

5. The process as claimed in one or more of claims 1 to 4, wherein the reaction is carried out in a dipolar-aprotic solvent, in particular dimethylformamide.

6. The process as claimed in one or more of claims 1 to 5, wherein the reaction is carried out at −20 to +40°C, preferably at −5 to +25°C.

7. The process as claimed in one or more of claims 1 to 6, wherein all the OH groups in the glycosyl fluoride used as the starting material are unsubstituted.

8. The process as claimed in one or more of claims 1 to 7, wherein the glycosyl fluoride used as the starting material has been partially acylated.

9. As new compounds, 2-acetamido-3,4,6-tri-0-benzyl-2-desoxy-α-D-glycopyranosyl fluoride, 2,3,4,6-tetra-0-n-butyl-α-D-glucopyranosyl fluoride and 2,3,4,6-tetra-0-n-octyl-α-D-glucopyranosyl fluoride.

# FIG.1

# FIG.2